# EUROPEAN PATENT APPLICATION

(11) **EP 2 413 125 A1**
(43) Date of publication of application: **01.02.2012**
(21) Application number: 10755864.5
(22) Date of filing: 10.03.2010
(51) Int. Cl.: G01N 1/28, B21D 51/18, C22C 16/00

(54) **ZIRCONIUM CRUCIBLE**

(30) Priority: 23.03.2009 JP 2009069440
(71) Applicant: JX Nippon Mining & Metals Corporation, Chiyoda-ku Tokyo 100-0004 (JP)
(72) Inventor: SAKAGUCHI Masahiro, Kitaibaraki-shi Ibaraki 319-1535 (JP); ENDO Ryosai, Kitaibaraki-shi Ibaraki 319-1535 (JP); TAKAHASHI Tomio, Kitaibaraki-shi Ibaraki 319-1535 (JP); SHINDO Yuichiro, Kitaibaraki-shi Ibaraki 319-1535 (JP)
(74) Representative: Hoarton, Lloyd Douglas Charles
(86) International application number: PCT/JP2010/053986
(87) International publication number: WO 2010/110064

(57) **Abstract**

Provided is a zirconium crucible for analytical use, wherein the purity excluding gas components is 3N or higher and the content of oxygen as a gas component is 500 mass ppm or less. In light of the recent analytical technology for which a fast and accurate measurement of high-purity materials is required; an object of the present invention is to inhibit the incorporation of impurities from a crucible by using a high-purity crucible, and provide a zirconium crucible for analytical use, wherein a two-stage separation/decomposition process is not required in the analysis of samples in which various types of oxides and metals such as sludge, bottom sediment samples and soil coexist, and the number of times that the crucible can be used is increased by improving the durability of high-purity zirconium metal.

## Description

### TECHNICAL FIELD

The present invention relates to a zirconium crucible for analytical use capable of increasing the number of times that the crucible can be used even in the analysis of samples in which various types of oxides and metals such as sludge, bottom sediment samples and soil coexist.

### BACKGROUND ART

In recent years, demands for quickly and accurately measuring high-purity materials are on the rise. Pursuant to the increase of such demands, there is a problem in that the measurement result differs depending on who or how skilled the analyst is, and reanalysis would often be performed in order to confirm the reliability of the initial analysis.
A sample for analysis is generally prepared by melting the sample with a flux. The process of melting the sample with a flux is usually based on a melting method such as carbonate (alkali) fusion, alkali hydroxide fusion, sodium peroxide fusion, or sodium hydrogensulfate fusion.

Among the above, sodium peroxide has strong oxidizing power, and is a favorable flux. Although an iron or nickel crucible is often used as the melting crucible in the foregoing case, it is necessary to take into consideration that the crucible will be severely affected.
In this sodium peroxide fusion, although the mixture proportion differs depending on the nature of the sample, sodium peroxide that is 5 to 10 times the sample amount is generally used (Non Patent Document 1). In addition, the heating temperature must also be changed depending on the sample, and this is decided entirely by experience.

Conventionally, a zirconium crucible was mainly used for determining the quantity of impurities contained in the analytical sample. In order to further reduce the lower quantitative limit, it is necessary to reduce contamination during the analysis as much as possible, and focus has been placed on reducing the amount of impurities that is eluted from the crucible.
Conventionally, although the quantitative value was sought by subtracting the blank of the crucible, variation in the blank depends largely on the skill of the analyst. Further, since a conventional zirconium crucible has a purity level of 99 wt% (2N), incorporation of impurities from the crucible causes the lower quantitative limit to become high, and this was insufficient for the analysis of recent high-purity samples.

There are not many Patent Documents that describe an analytical means to handle the foregoing high-purity materials. To introduce some Documents that may be of reference, for instance, there is technology that relates to the method of adjusting a sample for performing qualitative and quantitative analysis of such sample, wherein the sample is placed on a metal foil and subject to thermolysis together with such metal foil to put it into solution (Patent Document 1). Nevertheless, this is a special method, and lacks versatility..
Further, disclosed is that a crucible to perform chemical analysis with use of alkali flux is a crucible for chemical analysis composed of Pt alloy or Pd alloy in which 5 to 90 wt% of Pd is added to Pt (Patent Document 2). Nevertheless, this technology is impractical since expensive crucible materials are used herein.

In addition, disclosed is a method of analyzing the rhodium content in a film by heating and melting a rhodium-ruthenium alloy plating film in a nickel crucible with sodium peroxide or potassium peroxide (Patent Document 3). Nevertheless, Patent Document 3 does not in any way disclose the purity of the crucible, It is therefore strongly assumed that the crucible of Patent Document 3 has a conventional purity level (2N level). Thus, there is a problem in that the lower quantitative limit is high due to the incorporation of impurities, and high-precision analysis cannot be performed.

In light of the above, the present inventors invented a zirconium crucible wherein the purity excluding gas components is 3N or higher and the content of carbon as a gas component is 100 mass ppm or less (refer to Patent Document 4), and demonstrated that this is effective for achieving durability against melting of alkali flux or the like in the course of subjecting a sample to alkali fusion. This contributed considerably to the improvement of a conventional zirconium crucible. Although the reduction of the carbon content was an effective method, due to the diversification of the objects to be analyzed; for instance, sludge, bottom sediment and soil, this method became insufficient in overcoming the foregoing problem.

Conventionally, a high-purity zirconium crucible was mainly used for determining the quantity of impurities contained in the analytical sample. This is because it is necessary to inhibit the elution of impurities from the crucible in order to reduce the lower quantitative limit, and high-purity zirconium was extremely suitable therefor.
The samples to be analyzed were conventionally metal materials and oxide materials, and the constituents were only metal (high-purity Cu, high-purity Co, etc.) or only oxide (Si or Al-based oxide, etc.). However, if the samples to be analyzed further include sludge, bottom sediment and soil, silicic acid and alumina become the main components and metals and organic matter coexist therewith (refer to Non Patent Document 2).

The general procedures for performing conventional analysis are as follows.
(1) Place the sample in the zirconium crucible.
(2) Add a flux, such as an alkali flux, in the crucible.
(3) Heat the crucible with a burner or a muffle furnace to melt the flux and sample.
(4) Transfer the sample into a PTFE beaker or the like.
(5) Add acid or the like.
(6) Heat the beaker to dissolve the sample.
(7) Transfer the sample into a volumetric flask.
(8) Add water until the liquid measure becomes a prescribed value.
(9) Measure the result with an ICP-AES or the like.

Upon analyzing the foregoing materials, the following decomposition/melting method was conventionally performed.
1) Metal materials: Acid digestion
2) Oxide materials: Alkali fusion method
3) Metal + oxide materials: Acid digestion + alkali fusion method
   When measuring the result with the foregoing ICP-AES, it is known that the measurement by decomposing the sample with acid digestion generally enables to decrease the lower quantitative limit and achieve stable measurement performance. Thus, although it is desirable to apply acid digestion to all analytical samples, there are cases where certain oxides will not melt even with acid digestion.

In light of the foregoing circumstances, for materials in which metal components are mixed or chemically combined with oxides, analysis was performed by melting the metal portions with acid digestion, subsequently filtering this to separate the oxides, and decomposing the oxides based on the alkali fusion method. This process corresponds to step 3) of foregoing paragraph [0009] which describes the two process steps of decomposition and melting, and is an extremely complicated process by which, in certain cases, the analysis time takes approximately 12 hours.
Thus, in order to shorten the analysis time, attempts have been made of using the existing high-purity zirconium crucible and subjecting the analytical material containing metals to the alkali fusion, but the reaction with metals becomes severe in the foregoing case, and there is a problem in that the crucible becomes perforated soon, and its application was difficult since the number of times that the crucible can be used was limited.

[Non Patent Document 1] "Analysis" Introductory Course, Issued in October 1979, "Reagent Used in Dissolution" Pages 648 to 655
[Non Patent Document 2] "Alkali Fusion Using High-purity Nickel and Zirconium Crucible" by Yukio Murai, Environmental Measurement Technology, Vol. 34, No. 12, Pages 53 to 57

[Patent Document 1] Japanese Laid-Open Patent Publication No. H10-38773
[Patent Document 2] Japanese Laid-Open Patent Publication No. H2-172540
[Patent Document 3] Japanese Laid-Open Patent Publication No. S58-48854
[Patent Document 4] Japanese Patent Application No. 2007-213690

### DISCLOSURE OF THE INVENTION

In light of the recent analytical technology for which a fast and accurate measurement of high-purity materials is required; an object of the present invention is to inhibit the incorporation of impurities from a crucible by using a high-purity crucible, and provide a zirconium crucible for analytical use, wherein a two-stage separation/decomposition process is not required in the analysis of samples in which various types of oxides and metals such as sludge, bottom sediment samples and soil coexist, and the number of times that the crucible can be used is increased by improving the durability of high-purity zirconium metal.

In order to achieve the foregoing object, the present invention provides:
1. A zirconium crucible for analytical use, wherein the purity excluding gas components is 3N or higher and the content of oxygen as a gas component is 500 mass ppm or less;
2. The zirconium crucible according to claim 1, wherein the oxygen content is 200 mass ppm or less;
3. The zirconium crucible according to claim 1, wherein the oxygen content is 100 mass ppm or less; and
4. The zirconium crucible according to any one of claims 1 to 3, wherein the crucible is manufactured based on deep drawing.

Moreover, it is also effective to simultaneously reduce the content of carbon as a gas component contained in the zirconium; specifically, the carbon content can be reduced to 100 mass ppm or less, preferably 50 mass ppm or less, and more preferably 10 mass ppm or less. It is also effective to reduce the average crystal grain size of zirconium as the crucible material to 500 µm or less, preferably 100 µm or less, and more preferably 10 µm or less.

### [Effect of the Invention]

By using a zirconium crucible, wherein the purity excluding gas components is 3N or higher and the content of oxygen as a gas component is 500 mass ppm or less; the present invention yields a superior effect of being able to perform the analysis of samples, in which various types of oxides and metals such as sludge, bottom sediment samples and soil coexist, without going through a two-stage separation/decomposition process, and to increase the number of times that the zirconium crucible can be used by improving the durability of high-purity zirconium body.
It is thereby possible to simultaneously inhibit the incorporation of impurities from the crucible, perform high-precision analysis, shorten the operation time, and mitigate the amount of sample to be used. Thus, the present invention is able to meet the demands of recent analytical technology for which a fast and accurate measurement of high-purity materials is required.

### BRIEF DESCRIPTION OF THE DRAWINGS

[Fig. 1] Fig. 1 is a diagram showing a state where the corrosion of the crucible has advanced and the bottom of the crucible is perforated.
[Fig. 2] Fig. 2 is a diagram showing a state where the corrosion has advanced in spots (approximately 10 spots) at the bottom of the crucible, and the bottom has been partially perforated.

### BEST MODE FOR CARRYING OUT THE INVENTION

As described above, it has been discovered that the reduction of the oxygen concentration in the Zr yields obvious effects against the corrosion of the Zr crucible in the decomposition process, based on the alkali fusion of systems in which various modes of oxides and metal components coexist. In particular, the occurrence of Zr corrosion differs considerably from 500 ppm. Especially, when a Zr crucible contains the oxygen content exceeding 1000 ppm, the corrosion will advance in one or two performances of the analytical work, and the crucible becomes perforated.
Meanwhile, the analytical work can be performed several times or more without any perforation caused by the corrosion if the oxygen content is 500 ppm or less. In particular, it was found that the crucible withstands the analytical work 20 times or more on an average when the oxygen content was reduced to 200 ppm or less, and withstands the analytical work over 40 times when the oxygen content was reduced to 100 ppm or less. The number of analytical works as referred to herein is in relation to the following analytical procedures by which the effect of the present invention was discovered, but it is possible to assume that similar effects can be relatively yielded for conventionally known crucibles which are used under similar severe decomposition conditions.

In the foregoing case, although it is difficult to provide a technical explanation as to why the existence of oxygen is a significant factor in the rapid corrosion (perforation) of the zirconium crucible, it is generally known that, when the alkali fusion is performed, the zirconium on the surface of the zirconium crucible is gradually ground away. If the oxygen content in the crucible is large, it is considered that small voids are generated internally since the density of zirconium will deteriorate. Consequently, when the alkali fusion is continued and reaches the voids, it is considered that the pitting corrosion of the zirconium crucible advances with the voids as the source.
Up to now, there was no conception that the rapid corrosion (perforation) of the zirconium crucible which appears as local pitting corrosion was influenced by oxygen, and even if a zirconium crucible was inevitably used in the decomposition process, based on the alkali fusion of systems in which oxides and metal components coexist, the only understanding was that it was an inadequate crucible.

Moreover, since the elimination of oxygen from zirconium requires the precision of refinement to be improved and there is a problem of increased costs in terms of the manufacturing process, there has been no concept of restricting (reducing) the amount of oxygen in the zirconium crucible. Nevertheless, as a result of considerably increasing the number of times that the zirconium crucible can be used, it was possible to improve the durability to a level of being able to sufficiently absorb the increased costs for manufacturing the crucible, and the present invention achieved an economical effect of cost reduction.

### (Manufacturing process of crucible)

The manufacturing process of the crucible is as follows.
As the method of reducing the oxygen in the zirconium, for example, the electron beam melting method described in Japanese Patent Application No. 2000-302392 (Japanese Laid-Open Patent Publication No. 2002-105552), which is an invention by the present applicant, can be used to reduce the oxygen. Although in the Examples of this Patent Document, the oxygen content is reduced to 120 ppm; it is possible to further reduce the oxygen content to 100 ppm or less by performing quality governing such as reduction of the amount of oxygen or oxides at the raw material stage or repetition of the electron beam melting process by the utilization of the technology of this Patent Document.
The oxygen-reduced Zr used in the present invention was melted in an inert gas atmosphere or a vacuum to obtain an ingot. The obtained Zr ingot was rolled and subject to deep drawing to manufacture a crucible with a diameter of 3 cm, height of 4 cm, thickness of 1 mm, and an internal volume of 20 cc.

The results of reducing the oxygen content of the zirconium crucible were as described above, but as a result of reducing the oxygen content of the Zr ingot as with the present invention, the processing characteristics were improved, and it became possible to manufacture the crucible by rolling the ingot and performing deep drawing thereto.
A conventional Zr ingot with a high oxygen content had inferior workability and, therefore, was processed into a crucible shape by way of cutting. Consequently, most of zirconium would be wasted, the manufacturing cost of the crucible was extremely expensive and the production yield was inferior. But the present invention also yielded an additional effect of cost reduction with respect to the foregoing point.

### (Analysis procedures)

The analysis procedures are as follows.
(1) Place 0.5 g of the sample in the zirconium crucible described in paragraph [0021].
(2) Add 5 g of alkali flux (Na₂O₂) in the crucible.
(3) Heat the crucible with a burner or a muffle furnace to melt the flux and sample.
(4) Transfer the sample into a PTFE beaker and add 50 ml of water.
(5) Add 20 ml of hydrochloric acid.
(6) Heat the beaker to dissolve the sample.
(7) Transfer the sample into a volumetric flask.
(8) Add water until the liquid measure becomes 250 ml.
(9) Measure the result with an ICP-AES.
Analysis was performed based on the foregoing procedures, and a series of procedures was repeated.

In order to enable high-precision analysis, it is also effective to reduce contamination from the crucible. For example, if a high-purity crucible of 4N or higher is used, there is hardly any problem in terms of analytical precision. Since an application for a high-purity Zr crucible has previously been filed (refer to Japanese Patent Application No. 2006-146971), it is also effective to use the high-purity zirconium crucible thereof.

Moreover, even with a (high-purity) Zr crucible with a low impurity content, if it has a large C content, the reduction in weight will similarly increase (refer to foregoing Patent Document 4). Although the high purification of the zirconium crucible is desired as a matter of course, restriction of the carbon content is also important. As a result of restricting the carbon content, it is possible to improve the analytical precision even with a 3N level crucible, and further increase the number of times that the crucible can be used.
Since zirconium comprises a hexagonal close-packed (HCP) structure and is easily oriented toward a specific plane, the level of elution will considerably differ depending on the crystal plane. In order to inhibit the foregoing elution, the crystal grain size can be reduced as much as possible, and thereby reduce any elution bias. Accordingly, in the present invention, it is possible to employ the foregoing technologies in combination.

### [Examples]

The present invention is now explained based on the Examples and Comparative Examples. The Examples merely illustrate a preferred example, and the present invention shall in no way be limited thereby. In other words, all modifications, other embodiments and modes covered by the technical spirit of the present invention shall be included in this invention.
Note that the Examples and Comparative Examples show the results of performing quantitative analysis to Fe, Cr, Ni, Mn, Cu and Al based on bottom sediment samples as shown in Non Patent Document 2.

### (Example 1)

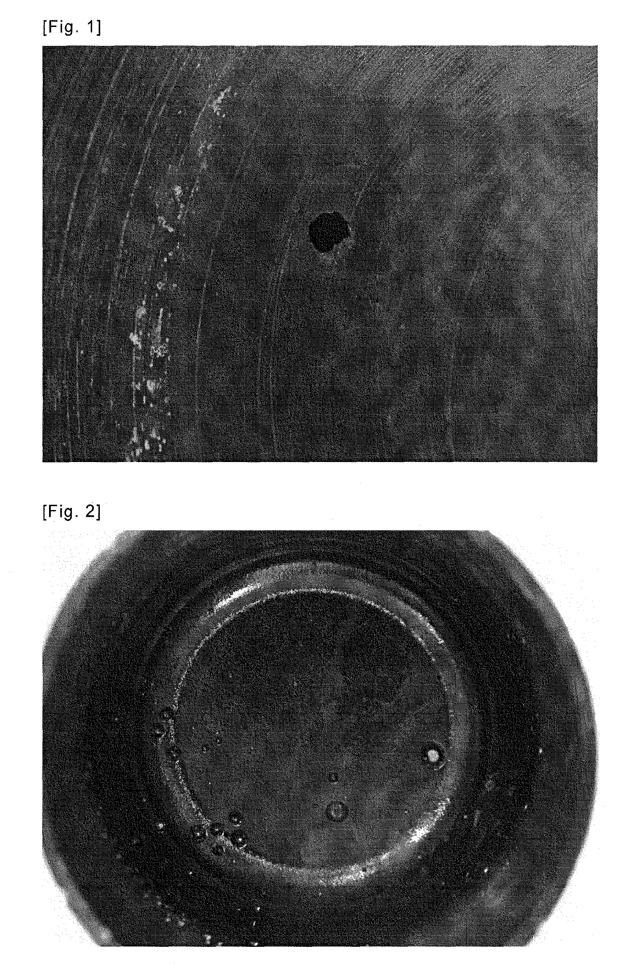

Based on the foregoing process, fifteen Zr crucibles in which the purity excluding gas components is 3N and the oxygen content is 80 mass ppm were prepared, and the foregoing analysis was repeated once a day. One of the Zr crucibles was perforated on the 41st analysis, another Zr crucible was perforated on the 54th analysis, and another Zr crucible was perforated on the 61st analysis and became unusable. Ultimately, it was possible to use the Zr crucibles up to 90 times on average.
The status of perforation is shown in Fig. 1 and Fig. 2. As shown in Fig. 1 and Fig. 2, corrosion did not occur evenly across the entire crucible, and numerous pitting corrosions can be observed. The results are also shown in Table 1 for ease of understanding.

**[Table 1]**

| | Oxygen content (mass ppm) | Average number of times crucible can be used |
|---|---|---|
| Example 1 | 80 | 110 (41, 54, 61, ···) |
| Example 2 | 180 | 75 |
| Example 3 | 350 | 35 |
| Example 4 | 450 | 20 |
| Comparative Example 1 | 700 | 5 |
| Comparative Example 2 | 1800 | 1.5 |
| Comparative Example 3 | 2500 | 0.8 |

### (Example 2)

Fifteen Zr crucibles in which the purity excluding gas components is 3N and the oxygen content is 180 ppm were prepared, and the foregoing analysis was repeated once a day. Upon similarly examining the number of times until the Zr crucibles became perforated and unusable, it was possible to use the Zr crucibles up to 75 times on average. The results are also shown in Table 1.

### (Example 3)

Fifteen Zr crucibles in which the purity excluding gas components is 3N and the oxygen content is 350 ppm were prepared, and the foregoing analysis was repeated once a day. Upon similarly examining the number of times until the Zr crucibles became perforated and unusable, it was possible to use the Zr crucibles up to 35 times on average. The results are also shown in Table 1.

### (Example 4)

Fifteen Zr crucibles in which the purity excluding gas components is 3N and the oxygen content is 450 ppm were prepared, and the foregoing analysis was repeated once a day. Upon similarly examining the number of times until the Zr crucibles became perforated and unusable, it was possible to use the Zr crucibles up to 20 times on average. The results are also shown in Table 1.

### (Comparative Example 1)

Fifteen Zr crucibles in which the purity excluding gas components is 3N and the oxygen content is 700 ppm were prepared, and the foregoing analysis was repeated once a day. Upon similarly examining the number of times until the Zr crucibles became perforated and unusable, it was possible to use the Zr crucibles only up to 5 times on average. The results are also shown in Table 1.

### (Comparative Example 2)

Fifteen Zr crucibles in which the purity excluding gas components is 3N and the oxygen content is 1800 ppm were prepared, and the foregoing analysis was repeated once a day. Upon similarly examining the number of times until the Zr crucibles became perforated and unusable, it was possible to use the Zr crucibles only up to 1.5 times on average. The results are also shown in Table 1.

### (Comparative Example 3)

Fifteen Zr crucibles in which the purity excluding gas components is 3N and the oxygen content is 2500 ppm were prepared, and the foregoing analysis was repeated once a day. Upon similarly examining the number of times until the Zr crucibles became perforated and unusable, it was possible to use the Zr crucibles only up to 0.8 times on average.

In both the Examples and Comparative Examples, recesses arise on the inner surface of the crucible, and such recesses become enlarged and penetrate the crucible, and thereby cause the crucible to be unusable. Although these recesses mainly arise randomly on the bottom surface of the crucible, they also occur at the side surface that is lower than the vicinity of the liquid level to which the analytical sample is constantly in contact.
After a recess arises, the crucible is often perforated after being subject to several more analyses.

### INDUSTRIAL APPLICABILITY

By using a zirconium crucible, wherein the purity excluding gas components is 3N or higher and the content of oxygen as a gas component is 500 mass ppm or less; the present invention yields a superior effect of being able to perform the analysis of samples, in which various types of oxides and metals such as sludge, bottom sediment samples and soil coexist, without going through a two-stage separation/decomposition process, and to increase the number of times that the zirconium crucible can be used by improving the durability of high-purity zirconium body.
It is thereby possible to inhibit the incorporation of impurities from the crucible, perform high-precision analysis, shorten the operation time, and mitigate the amount of sample to be used. Thus, the present invention is able to meet the demands of recent analytical technology which require fast and accurate measurement of high-purity materials, and is effective as a zirconium crucible for analytical use.

## Claims

1. A zirconium crucible for analytical use, wherein the purity excluding gas components is 3N or higher and the content of oxygen as a gas component is 500 mass ppm or less.

2. The zirconium crucible according to claim 1, wherein the oxygen content is 200 mass ppm or less.

3. The zirconium crucible according to claim 1, wherein the oxygen content is 100 mass ppm or less.

4. The zirconium crucible according to any one of claims 1 to 3, wherein the crucible is manufactured based on deep drawing.
